Europäisches Patentamt

**European Patent Office**

Office européen des brevets

(11) Veröffentlichungsnummer: **0 045 849**
A1

(12) **EUROPÄISCHE PATENTANMELDUNG**

(21) Anmeldenummer: 81105281.0

(22) Anmeldetag: 08.07.81

(51) Int. Cl.³: **A 61 M 25/00**
**A 61 M 5/14**

(30) Priorität: 13.08.80 DE 3030579

(43) Veröffentlichungstag der Anmeldung:
17.02.82 Patentblatt 82/7

(84) Benannte Vertragsstaaten:
AT BE CH DE FR GB IT LI LU NL SE

(71) Anmelder: Intermedicat GmbH
Gerliswilstrasse 45
CH-6020 Emmenbrücke(CH)

(72) Erfinder: Werner, Hans-Theo, Ing. grad.
Parkstrasse 1
D-3501 Edermünde-Grifte(DE)

(72) Erfinder: Herlitze, Gerhard, Ing. grad.
Binsdorfer Strasse 4
D-3507 Baunatal 7(DE)

(74) Vertreter: von Kreisler, Alek, Dipl.-Chem. et al,
Deichmannhaus am Hauptbahnhof
D-5000 Köln 1(DE)

(54) Venenverweilkatheter.

(57) Der Venenverweilkatheter besteht aus einer Metallkanüle (4), die an ihrem vorderen Ende einen Anschliff (7) und an
ihrem rückwärtigen Ende ein Griffstück (5) aufweist. Die
Metallkanüle (4) ist in einen Kapillarschlauch (1) eingeschoben, aus dessen vorderem Ende das Ende der Metallkanüle
(4) herausragt. Um zu erreichen, daß der Überstand der
Metallkanüle (4) am vorderen Ende des Kapillarschlauches
(1) einen definierten Wert einnimmt, ist das mit der Metallkanüle (4) verbundene Griffstück (5) relativ zu dem mit dem
Kapillarschlauch (1) verbundene Griffstück (2) in Längsrichtung verstellbar. Diese Verstellbarkeit wird z.B. durch einen
Keilring (12) erreicht, der unterschiedliche Drehstellungen
annehmen kann und nach seiner Einstellung fixiert wird.

FIG. 1

EP 0 045 849 A1

VON KREISLER   SCHÖNWALD   EISHOLD   FUES 45849
VON KREISLER   KELLER   SELTING   WERNER

PATENTANWÄLTE

Dr.-Ing. von Kreisler † 1973
Dr.-Ing. K. Schönwald, Köln
Dr.-Ing. K. W. Eishold, Bad Soden
Dr. J. F. Fues, Köln
Dipl.-Chem. Alek von Kreisler, Köln
Dipl.-Chem. Carola Keller, Köln
Dipl.-Ing. G. Selting, Köln
Dr. H.-K. Werner, Köln

DEICHMANNHAUS AM HAUPTBAHNHOF
D-5000 KÖLN 1

Sg/En

B. Braun Melsungen AG

Carl-Braun-Str. 1

3508 Melsungen

Venenverweilkatheter

Die Erfindung betrifft einen Venenverweilkatheter, mit einem über eine Metallkanüle gezogenen Kapillarschlauch, dessen proximales Ende in der Nähe eines am vorderen Ende der Metallkanüle vorgesehenen Anschliffes angeordnet ist, und mit jeweils einem mit dem rückwärtigen Ende des Kapillarschlauches verbundenen ersten Griffstück, durch das die Metallkanüle hindurchgeht, und einem mit dem rückwärtigen Ende der Metallkanüle verbundenen zweiten Griffstück, wobei beide Griffstücke relativ zueinander verschiebbar sind und die Verschiebebewegung durch Anschlagflächen begrenzt ist.

Um Flüssigkeiten in das Blutsystem eines Patienten zu übertragen, wird ein Venenzugang benötigt, um die Vene an eine Kanüle bzw. einen Katheter anzuschließen. Zu diesem Zweck muß die Vene punktiert werden. Bekannt sind

Venenverweilkatheter aus einem flexiblen Kapillarschlauch, dessen proximales Ende in die punktierte Vene eingeführt wird. Zum Punktieren der Vene und zum Einführen des Kapillarschlauches wird in den Kapillarschlauch eine Metallkanüle mit geschliffener Spitze eingeschoben. Mit der in den Kapillarschlauch eingesetzten Metallkanüle, die an ihrem aus dem Kapillarschlauch herausragenden vorderen Ende eine schräge Schneidkante aufweist, werden die Haut und das betreffende Blutgefäß punktiert. Blutrückfluß am rückwärtigen Ende der Metallkanüle zeigt die erfolgreiche Punktion des Blutgefäßes an. Danach wird die Metallkanüle zusammen mit dem Kapillarschlauch weiter vorgeschoben, bis der Kapillarschlauch das Blutgefäß erreicht. Anschließend wird die Metallkanüle zurückgezogen, so daß nur noch der Kapillarschlauch an das Blutgefäß angeschlossen ist. Bei dünnlumigen Blutgefäßen ist der Abstand zwischen dem Schliffende (der schrägen Schneidkante am vorderen Ende der Metallkanüle) und dem Anfang des Kapillarschlauches von entscheidender Bedeutung. Ist dieser Abstand zu groß, so wird bei derartigen Gefäßen parapunktiert. Dies bedeutet, daß der Kapillarschlauch auf der Metallkanüle durch das Blutgefäß hindurchgeschoben wird oder daß er vor dem Blußgefäß im Gewebe endet. Für die Praxis ist es daher wichtig zu erreichen, daß das vordere Ende des Kapillarschlauches sich so nahe wie möglich an dem Anschliff der Metallkanüle befindet.

Die verschiedenen Fertigungsverfahren bedingen unterschiedliche Toleranzen beim Herstellungsprozeß und führen dazu, daß am fertigen Produkt der Abstand zwischen dem Schliffende der Metallkanüle und dem vorderen Ende des Kapillarschlauches variiert. Um diesen Abstand möglichst klein und konstant zu machen, ist es bekannt, die Längen der einzelnen Kapillarschläuche vor ihrer Montage zu messen und durch geeignete Auswahl der Kapillarschläuche eine Anpassung an die jeweilige Metallkanüle zu bewirken. Ferner geschieht die Längenanpassung durch sogenanntes Strippen, d.h. Zusammenschieben oder Auseinanderziehen des Kapillarschlauches. Schließlich ist es bekannt, die Längenabstimmung während des Montageprozesses durch Verschieben der Metallkanüle vorzunehmen.

Der Erfindung liegt die Aufgabe zugrunde, einen Venenverweilkatheter der eingangs genannten Art zu schaffen, der die Möglichkeit einer Längenkorrektur des Kapillarschlauches bzw. der gegenseitigen Abstimmung der Lagen der vorderen Enden von Metallkanüle und Kapillarschlauch mit einfachen technischen Mitteln bietet.

Zur Lösung dieser Aufgabe ist gemäß einer ersten Variante der Erfindung die Anschlagfläche mindestens eines der Griffstücke in Längsrichtung verstellbar.

Durch die Verstellung der Anschlagfläche eines Griffstückes werden nicht nur die Axialpositionen der Griffstücke zueinander justiert, sondern auch die relativen Axialpositionen von Kapillarschlauch und Metallkanüle. Durch eine relativ einfache Verstelleinrichtung wird erreicht, daß der Abstand zwischen dem Schliffende der Metallkanüle und dem vorderen Ende des Kapillarschlauches bei jedem einzelnen Gerät individuell justiert und gewünschtenfalls auf Null gebracht werden kann.

Die zusammenwirkenden Anschlagflächen, die die Relativpositionen von Metallkanüle und Kapillarschlauch festlegen, müssen nicht notwendigerweise vollflächig aneinanderliegen. Es genügt, wenn sie sich an mindestens einer Stelle berühren, da hierdurch bereits die Verschiebebewegung begrenzt wird. Während des Punktierens werden. die beiden Griffstücke mit der Hand gegeneinandergedrückt, so daß die Anschlagflächen (an mindestens einem Punkt) gegeneinanderstoßen. Infolge der Verstellbarkeit der Anschlagflächen kann die Justierung so vorgenommen werden, daß in diesem Zustand die gewünschte räumliche Zuordnung von Metallkanüle und Kapillarschlauch erreicht ist.

Bei einer bevorzugten Ausführungsform der Erfindung weist eines der Griffstücke ein drehbares Ausgleichsstück mit mindestens einer Stirnfläche auf, die eine Schrägfläche eines der Griffstücke abstützt. Das Ausgleichsstück mit der schrägen Stirnfläche bildet ein Keilstück, dessen Abstützpunkt bzw. dessen wirksame Länge durch Verdrehung verändert werden kann. Nach Beendigung der Justierung des Ausgleichsstücks kann das Ausgleichstück an dem Griffstück z.B. durch thermische Punktschweißung, Kleben o.dgl. fixiert werden.

Vorzugsweise umgibt das Ausgleichsstück die Metallkanüle koaxial.

Bei einer alternativen Ausführungsform der ersten Variante der Erfindung weist eines der Griffstücke einen Gewindeansatz auf, auf dem ein durch Drehung axial verstellbarer Gewindering sitzt, an dem sich die verstellbare Anschlagfläche befindet.

0045849

- 5 -

Gemäß einer zweiten Variante der Erfindung ist das rückwärtige Ende der Metallkanüle oder des Kapillarschlauches in dem zugehörigen Griffstück verschiebbar und in einer wählbaren Stellung fixierbar. Vorzugsweise sind zwei konisch asymmetrisch angeordnete Stege vorgesehen, die gleichzeitig zur Befestigung einer Schutzkappe benutzt werden können und so angeordnet sind, daß bei aufgeschobenem Kapillarschlauch zunächst ein maximaler Abstand zwischen dem Schliffende der Metallkanüle und dem Anfang des Kapillarschlauches gegeben ist. Durch Einleiten mechanischer oder thermischer Impulse in die Anbindung der Stege an das Griffstück der Metallkanüle verändert sich der Öffnungswinkel der Stege, so daß sich der Kapillarschlauch praktisch stufenlos auf den gewünschten Abstand einstellen läßt.

Eine weitere Möglichkeit der Veränderung des Abstandes besteht darin, in einem Luftspalt zwischen den Griffstücken von Metallkanüle und Kapillarschlauch einen verformbaren Abstandhalter anzuordnen, der die Anschlagfläche bildet.

Bei der Punktion ist es erforderlich, daß der an der Metallkanüle befindliche Schliff nach oben zeigt und mit an den Griffstücken vorgesehenen Griffflächen fluchtet. Die beiden Griffstücke sollten daher verdrehungssicher zueinander fixiert sein, damit der Schliff stets in die gleiche Richtung zeigt wie die Griffplatten. Zu diesem Zweck kann eine überlappende Verdrehsicherung vorgesehen sein. Um zu verhindern, daß die Metallkanüle versehentlich um 180° verdreht eingesetzt wird, kann die Verdrehsicherung asymmetrisch ausgeführt werden.

Im folgenden werden unter Bezugnahme auf die Figuren einige Ausführungsbeispiele der Erfindung näher erläutert.

Es zeigen:

Figur 1 eine Seitenansicht einer ersten Ausführungsform des Venenverweilkatheters, teilweise aufgeschnitten, in der einen Extremstellung der Metallkanüle,

Figur 2 das Gerät nach Figur 1 im justierten Zustand,

Figur 3 ein Ausführungsbeispiel, bei dem das Ausgleichsstück an dem Griffstück des Kapillarschlauchs angebracht ist,

Figur 4 ein Ausführungsbeispiel, bei dem das Ausgleichsstück an dem Griffstück der Metallkanüle gelagert ist und zwei schräge Stirnflächen aufweist,

Figur 5 ein Ausführungsbeispiel mit Gewindering,

Figur 6 ein erstes Ausführungsbeispiel der zweiten Variante mit thermischer Fixierung der Metallkanüle in dem zweiten Griffstück,

Figur 7 ein weiteres Ausführungsbeispiel der zweiten Variante,

Figur 8 ein Ausführungsbeispiel, bei dem die Anschlagfläche durch Veränderung des Öffnungswinkels von Segmenten verändert wird,

Figur 9 ein Ausführungsbeispiel, bei dem der Abstand der Anschlagflächen durch verformbare Elemente veränderbar ist, und

Figur 10 einen Querschnitt entlang der Linie X-X von Figur 9 zur Verdeutlichung der Verdrehsicherung.

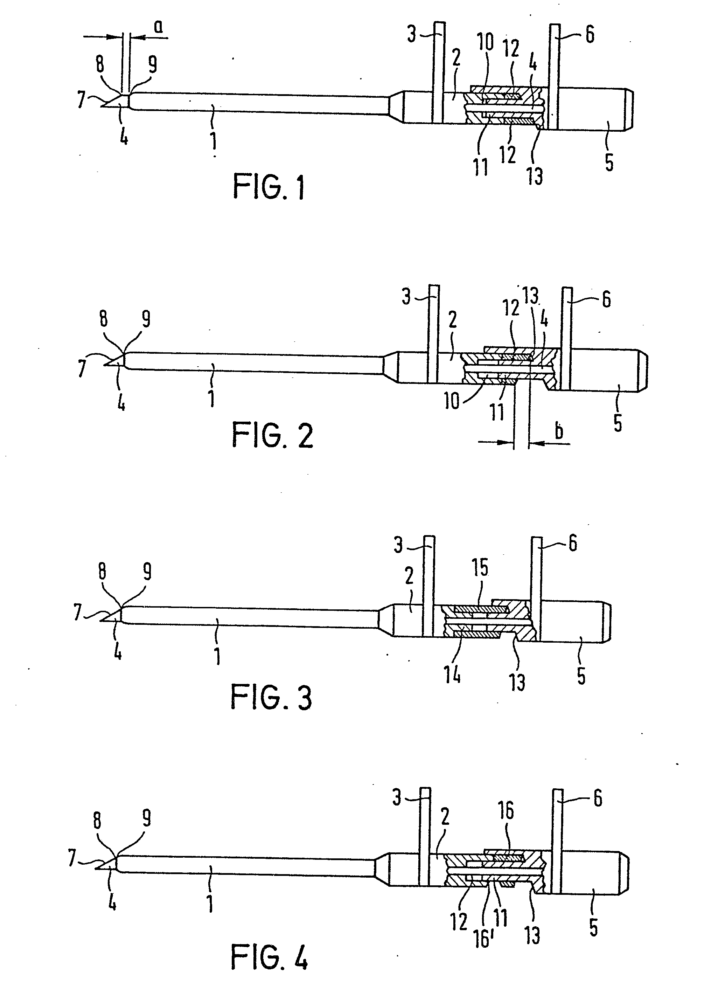

Der in den Figuren 1 und 2 dargestellte Venenverweilkatheter weist einen aus Kunststoff bestehenden Kapillarschlauch 1 auf, dessen rückwärtiges Ende an einem Griffstück 2 befestigt ist, von dem eine Griffplatte 3 absteht. In den Kapillarschlauch 1 ist durch das Griffstück 2 hindurch die Metallkanüle 4 eingeschoben. Am proximalen Ende der Stahlkanüle 4 befindet sich der schräge Schliff 7. Der maximale Abstand zwischen dem Schliffauge 8 und dem vorderen Ende 9 des Kapillarschlauches ist in der Zeichnung mit a bezeichnet. Am rückwärtigen Ende der Metallkanüle 4 ist das zweite Griffstück 5 befestigt, das relativ zu dem ersten Griffstück 2 verschiebbar ist und ebenfalls eine radial abstehende Griffplatte 6 aufweist.

An dem rückwärtigen Ende des ersten Griffstückes 2 befindet sich eine die Metallkanüle 4 umgebende Bohrung 10, in die ein ringförmiger Ansatz 11 des zweiten Griffstücks hineinragt. Auf dem Ansatz 11 ist ein drehbarer Keilring 12 gelagert, der sich mit seiner vorderen geraden Stirnfläche am hinteren Ende des ersten Griffstücks 2 abstützt und dessen abgeschrägte rückwärtige Stirnseite an einer Schrägfläche 13 des zweiten Griffstückes 5 anliegt.

Wenn der Abstand a in Figur 1 auf Null reduziert werden soll, wird der das Ausgleichsstück bildende Keilring 12 gedreht. Da die beiden Griffstücke 2 und 5 relativ zueinander nicht verdreht werden können, drückt die keilförmige Stirnseite des Keilringes 12 die Keilfläche 13 des zweiten Griffstückes 5 zurück, wodurch gleichzeitig die Metallkanüle 4 in dem Kapillarschlauch 1 zurückgezogen wird. Wenn das Maß der Zurückziehung der Metallkanüle einen Wert b erreicht hat, der dem Abstand a (Figur 1) entspricht, ist die erforderliche Übereinstimmung erreicht.

Figur 3 zeigt eine Venenverweilkanüle, die weitgehend derjenigen der Figuren 1 und 2 entspricht. Der Keilring 15 ist in Figur 3 jedoch nicht auf einem Ansatz des zweiten Griffstückes 5 gelagert, sondern auf einem Ansatz 14 des ersten Griffstückes 2.

Das Ausführungsbeispiel der Figur 4 entspricht demjenigen der Figuren 1 und 2, jedoch ist der Keilring 16 nicht nur an einer, sondern an seinen beiden Stirnseiten abgeschrägt. Dies ermöglicht einen größeren Verstellbereich bzw. bei gleichbleibendem Verstellbereich geringere Längenabmessungen des verwendeten Keilringes. Mit der vorderen schrägen Stirnseite des Keilringes 16 wirkt eine Schrägfläche 16' am rückwärtigen Ende des ersten Griffstückes 2 zusammen. Die Schrägflächen 13 und 16' haben jeweils den gleichen Schrägwinkel wie die zugehörigen Stirnseiten des Ausgleichsstückes 16. Die Schrägflächen und die Stirnseiten liegen daher nur im Falle des kleinsten Abstandes zwischen den Griffstücken 2 und 5 vollflächig gegeneinander.

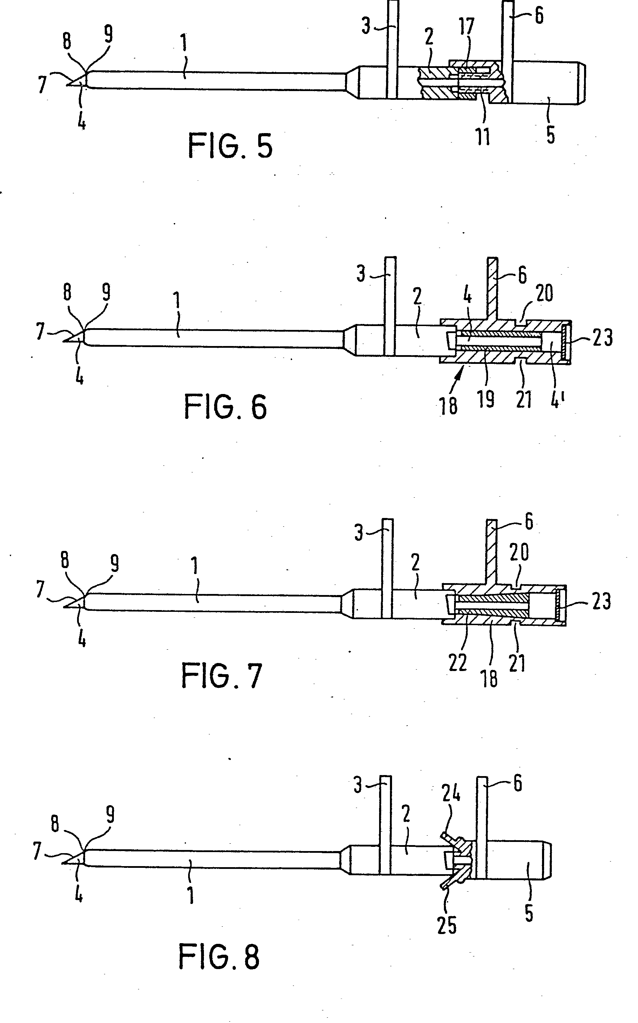

Bei dem Ausführungsbeispiel von Figur 5 ist am Ansatz 11, der von dem zweiten Griffstück 5 aus nach vorne vorsteht, ein Gewinde vorgesehen und auf dieses Gewinde ist ein Gewindering 17 aufgeschraubt, dessen vordere Stirnseite die Anschlagfläche für die rückwärtige Stirnseite des ersten Griffstücks 2 bildet. Durch Drehen des Gewinderinges 17 kann dessen axiale Stellung und damit auch die Position der Anschlagfläche verändert werden.

Figur 6 zeigt einen Venenverweilkatheter gemäß der zweiten Variante der Erfindung. Hierbei ist das rückwärtige Ende der Metallkanüle 4 mit einer in dem zweiten Griffstück 18 axial verschiebbaren Buchse 19 fest verbunden. Nach dem Einstellen des Abstandes a = 0 wird die Buchse 19 in dem Griffstück 18 fixiert. Zu diesem Zweck sind an dem aus Kunststoff bestehenden Griffstück 18 Einkerbungen 20, 21 vorgesehen, die mit einem geeigneten Werkzeug eine thermische oder mechanische Fixierung mit der Buchse 19 ermöglichen. Das rückwärtige Ende des Kanales 4' für die Metallkanüle im zweiten Griffstück 18 ist durch eine hydrophobierte Folie 23 verschlossen, die den Blutaustritt bei Punktion verhindert.

Figur 7 zeigt einen Venenverweilkatheter gemäß Figur 6 mit einer in dem zweiten Griffstück 18 axial verschiebbaren konischen Buchse 22, die mit der Metallkanüle 4 fest verbunden ist.

Figur 8 zeigt einen Venenverweilkatheter, bei dem der Abstand a = 0 durch Veränderung des Öffnungswinkels von Zungen 24, 25 eingestellt wird, die von dem zweiten Griffstück 5 in Richtung auf das erste Griffstück 2 schräg nach außen abstehen und bei unterschiedlichen Öffnungswinkeln unterschiedliche Anschlagflächen bilden.

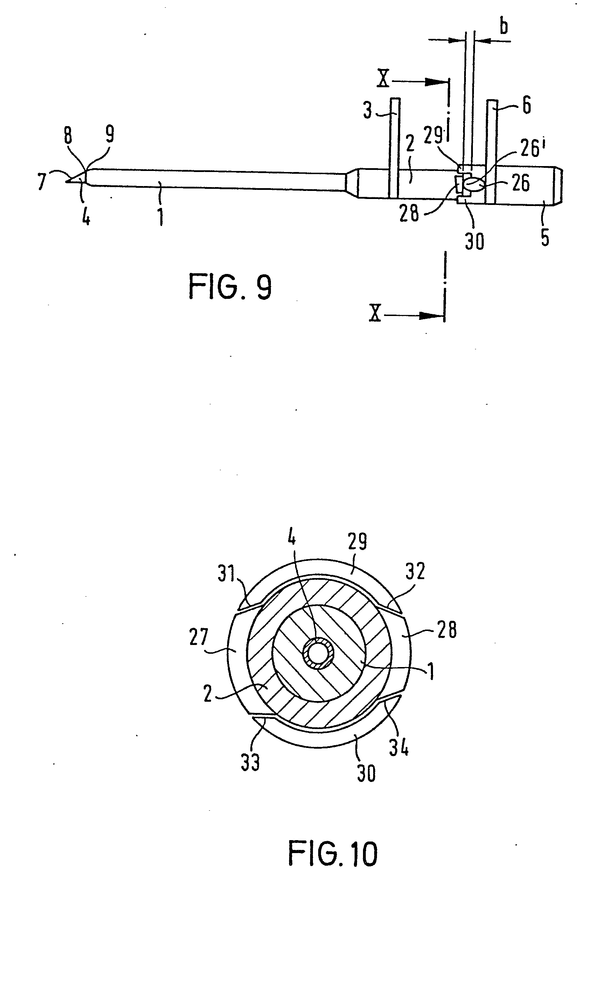

Bei dem Ausführungsbeispiel von Figur 9 befindet sich an dem zweiten Griffstück 5 eine verformbare Fläche 26, deren Vorderseite 26' gegen die rückwärtige Stirnseite der Nocken 27, 28 des ersten Griffstücks 2 stößt. Durch geeignete Verformung der Fläche 26 kann die Lage der Anschlagfläche 26' verändert werden.

Figur 10 zeigt einen Querschnitt durch Figur 9. Als Verdrehungssicherung zwischen dem Griffstück 2 und dem Griffstück 5 dienen die zwischen die Nocken 27 und 28 ragenden Segmente 29, 30 des zweiten Griffstücks 5. Die Begrenzungsflächen 31 und 32 des Segmentes 29 sind zu den Begrenzungsflächen 33 und 34 des Segmentes 30 asymmetrisch gestaltet, um ein unbeabsichtigtes Verdrehen um 180° auszuschließen.

Ansprüche

1. Venenverweilkatheter, mit einem über eine Metallkanüle gezogenen Kapillarschlauch, dessen proximales Ende in der Nähe eines am vorderen Ende der Metallkanüle vorgesehenen Anschliffes angeordnet ist, und mit jeweils einem mit dem rückwärtigen Ende des Kapillarschlauches verbundenen ersten Griffstück, durch das die Metallkanüle hindurchgeht, und einem mit dem rückwärtigen Ende der Metallkanüle verbundenen zweiten Griffstück, wobei beide Griffstücke relativ zueinander verschiebbar sind und die Verschiebebewegung durch Anschlagflächen begrenzt ist, d a d u r c h   g e k e n n z e i c h n e t, daß die Anschlagfläche mindestens eines der Griffstücke (2, 5) in Längsrichtung verstellbar ist.

2. Venenverweilkatheter nach Anspruch 1, dadurch gekennzeichnet, daß eines der Griffstücke (2, 5) ein drehbares Ausgleichsstück (12, 15, 16) mit mindestens einer schrägen Stirnfläche aufweist, die eine Schrägfläche (13) eines der Griffstücke (2, 5) abstützt.

3. Venenverweilkatheter nach Anspruch 2, dadurch gekennzeichnet, daß beide Stirnflächen des Ausgleichsstückes (16) abgeschrägt sind und mit je einer Schrägfläche (13, 16') eines der Griffstücke (5, 2) zusammenwirken.

4. Venenverweilkatheter nach einem der Ansprüche 1 bis 3, dadurch gekennzeichnet, daß das Ausgleichsstück (12, 15, 16, 17) die Metallkanüle (4) koaxial umgibt.

5. Venenverweilkatheter nach einem der Ansprüche 1 bis 4, dadurch gekennzeichnet, daß eines der Griffstücke (2, 5) einen Gewindeansatz (11) aufweist, auf dem ein durch Drehung axial verstellbarer Gewindering (17) sitzt, an dem sich die verstellbare Anschlagfläche befindet.

6. Venenverweilkatheter nach Anspruch 1, dadurch gekennzeichnet, daß eines der Griffstücke (2, 5) schräg abstehende Segmente (24, 25) aufweist, deren Anstellwinkel veränderbar ist und die mit der Anschlagfläche des anderen Griffstückes zusammenwirken.

7. Venenverweilkatheter nach Anspruch 1, dadurch gekennzeichnet, daß eines der Griffstücke (2, 5) verformbare Elemente (26) aufweist, die die Anschlagfläche bilden.

8. Venenverweilkatheter, mit einem über eine Metallkanüle gezogenen Kapillarschlauch, dessen proximales Ende in der Nähe eines am vorderen Ende der Metallkanüle vorgesehenen Anschliffes angeordnet ist, und mit jeweils einem mit dem rückwärtigen Ende des Kapillarschlauches verbundenen ersten Griffstück, durch das die Metallkanüle hindurchgeht, und einem mit dem rückwärtigen Ende der Metallkanüle verbundenen zweiten Griffstück, wobei beide Griffstücke relativ zueinander verschiebbar sind und die Verschiebebewegung durch Anschlagflächen begrenzt ist, dadurch gekennzeichnet, daß das rückwärtige Ende der Metallkanüle (4) oder des Kapillarschlauches (1) in dem zugehörigen Griffstück (2, 5) verschiebbar und in einer wählbaren Stellung fixierbar ist (Figuren 6 und 7).

FIG. 1

FIG. 2

FIG. 3

FIG. 4

0045849

FIG. 5

FIG. 6

FIG. 7

FIG. 8

0045849

FIG. 9

FIG.10

Europäisches
Patentamt

**EUROPÄISCHER RECHERCHENBERICHT**

Nummer der Anmeldung

EP 81 10 5281

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| A | US - A - 3 774 606 (NORTON) <br><br> * Spalte 1, Zeilen 60 bis 68; Spalte 2, Zeilen 1 bis 28; Figuren * <br><br> -- | 1 |
| A | FR - A - 2 067 542 (HENKIN) <br><br> * Seite 6, Zeilen 27 bis 37; Figuren 1 bis 5 * <br><br> ------ | 1 |

**KLASSIFIKATION DER ANMELDUNG (Int. Cl.³)**

A 61 M 25/00
5/14

**RECHERCHIERTE SACHGEBIETE (Int. Cl.³)**

A 61 M

**KATEGORIE DER GENANNTEN DOKUMENTE**

X: von besonderer Bedeutung

A: technologischer Hintergrund

O: nichtschriftliche Offenbarung

P: Zwischenliteratur

T: der Erfindung zugrunde liegende Theorien oder Grundsätze

E: kollidierende Anmeldung

D: in der Anmeldung angeführtes Dokument

L: aus andern Gründen angeführtes Dokument

&: Mitglied der gleichen Patentfamilie, übereinstimmendes Dokument

Der vorliegende Recherchenbericht wurde für alle Patentansprüche erstellt.

| Recherchenort | Abschlußdatum der Recherche | Prüfer |
|---|---|---|
| Den Haag | 25.11.1981 | VANRUNXT |

EPA form 1503.1   06.78